(19) Europäisches Patentamt
European Patent Office
Office européen des brevets

(11) **EP 2 504 044 B2**

(12) ## NEUE EUROPÄISCHE PATENTSCHRIFT
Nach dem Einspruchsverfahren

(45) Veröffentlichungstag und Bekanntmachung des Hinweises auf die Entscheidung über den Einspruch:
**04.10.2023 Patentblatt 2023/40**

(45) Hinweis auf die Patenterteilung:
**27.01.2016 Patentblatt 2016/04**

(21) Anmeldenummer: **10790889.9**

(22) Anmeldetag: **16.11.2010**

(51) Internationale Patentklassifikation (IPC):
**A61M 1/34** (2006.01)

(52) Gemeinsame Patentklassifikation (CPC):
**A61M 1/342; A61M 1/16; A61M 1/34; A61M 1/3403; A61M 1/341; A61M 1/3413; A61M 1/3434; A61M 1/3635;** A61M 2205/3334

(86) Internationale Anmeldenummer:
**PCT/EP2010/006981**

(87) Internationale Veröffentlichungsnummer:
**WO 2011/063906 (03.06.2011 Gazette 2011/22)**

(54) **EXTRAKORPORALE BLUTBEHANDLUNGSVORRICHTUNG MIT EINER EINRICHTUNG ZUM REGELN DER ZUFUHR VON SUBSTITUAT**

EXTRACORPOREAL BLOOD TREATMENT DEVICE COMPRISING A UNIT FOR REGULATING THE SUPPLY OF SUBSTITUATE

DISPOSITIF DE TRAITEMENT EXTRACORPOREL DU SANG COMPRENANT UN DISPOSITIF DE RÉGULATION DE L'ALIMENTATION EN SOLUTION DE SUBSTITUTION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.11.2009 DE 102009055995**

(43) Veröffentlichungstag der Anmeldung:
**03.10.2012 Patentblatt 2012/40**

(73) Patentinhaber: **Fresenius Medical Care Deutschland GmbH**
**61352 Bad Homburg v.d.H. (DE)**

(72) Erfinder:
• **KOPPERSCHMIDT, Pascal**
**97456 Dittelbrunn (DE)**
• **GAGEL, Alfred**
**96123 Litzendorf (DE)**

(74) Vertreter: **Oppermann, Frank**
**OANDO Oppermann & Oppermann**
**Wilhelminenstrasse 1a**
**65193 Wiesbaden (DE)**

(56) Entgegenhaltungen:
WO-A1-01/76661      WO-A1-2008/135193
WO-A1-2008/135193   WO-A1-2009/080258
DE-T2- 60 030 460   DE-T2- 60 037 408
US-A1- 2002 023 880 US-A1- 2002 174 721
US-A1- 2006 157 408 US-A1- 2007 108 128

• "Hagen?Poiseuille equation", Wikipedia, 2016, Retrieved from the Internet: URL:https://en.wikipedia.org/wiki/Hagen%E2%80%93Poiseuille_equation
• PRIES et al.: "Microvascular blood viscosity in vivo and the endothelial surface layer", Am J Physiol Heart Circ Physiol, vol. 289, no. 6, August 2005 (2005-08), pages H2657-H2664,

**EP 2 504 044 B2**

**Beschreibung**

Die Erfindung betrifft eine Vorrichtung zur extrakorporalen Blutbehandlung, die einen durch eine semipermeable Membran in eine Blutkammer und eine Dialysierflüssigkeitskammer unterteilten Dialysator und eine Einrichtung zum Zuführen von Substituat aufweist, wobei die extrakorporale Blutbehandlungvorrichtung über eine Einrichtung zum Regeln der Zufuhr von Substituat verfügt.

Zur Entfernung von hampflichtigen Substanzen und zum Flüssigkeitsentzug werden beim chronischen Nierenversagen verschiedene Verfahren zur apparativen Blutreinigung bzw. Blutbehandlung eingesetzt. Bei der Hämodialyse (HD) wird das Blut des Patienten in einem extrakorporalen Blutkreislauf durch die Blutkammer eines von einer semipermeablen Membran in die Blutkammer und eine Dialysierflüssigkeitskammer unterteilten Dialysators geleitet, während die Dialysierflüssigkeitskammer von einer Dialysierflüssigkeit durchströmt wird. Über die Membran des Dialysators findet im Wesentlichen ein diffuser Stoffaustausch statt. Bei der Hämofiltration (HF) wird die Dialysierflüssigkeitskammer nicht von Dialysierflüssigkeit durchströmt. Es findet nur ein konvektiver Stoffaustausch statt. Eine Kombination aus beiden Verfahren ist die Hämodiafiltration (HDF).

Die dem Patienten bei der Hämofiltration (HF) oder Hämodiafiltration (HDF) über die semipermeable Membran des Dialysators entzogene Menge an Flüssigkeit wird dem Patienten während der Blutbehandlung wieder als Substituat zugeführt, das entweder gebrauchsfertig bereitgestellt wird oder während der Blutbehandlung aus der Dialysierflüssigkeit gewonnen wird. Das Substituat wird dem extrakorporalen Blutkreislauf stromauf und/oder stromab des Dialysators zugeführt. Die Zufuhr von Substituat stromauf des Dialysators wird als Prädilution und stromab des Dialysators als Postdilution bezeichnet. Als Substituatrate wird diejenige Menge an Substituat bezeichnet, die in einem bestimmten Zeitraum dem im extrakorporalen Blutkreislauf strömenden Blut zugeführt wird.

Zur Bilanzierung von frischer und verbrauchter Dialysierflüssigkeit, die in die bzw. aus der Dialysierflüssigkeitskammer des Dialysators strömt; finden bei den bekannten Blutbehandlungsvorrichtungen Bilanziersysteme Verwendung. Die Bilanzierung von frischer und verbrauchter Dialysierflüssigkeit stellt sicher, dass dem Patienten keine Flüssigkeit oder nur eine bestimmte Menge an Flüssigkeit zugeführt oder entzogen wird.

Die Ultrafiltrationsrate, mit der dem Patienten Flüssigkeit entzogen wird, ist von dem Transmembrandruck TMP abhängig, der als der Druckunterschied zwischen dem mittleren, blutseitigen, und dem mittleren, dialysatseitigen Druck am Dialysator definiert ist. Verfahren und Vorrichtungen zum Bestimmen des Transmembrandrucks sind allgemein bekannt. Die EP 0 212 127 A1 und WO 2009/ 080258 A1 beschreiben beispielsweise eine Vorrichtung zur Bestimmung des Transmembrandrucks.

Neben dem Transmembrandruck ist für eine extrakorporale Blutbehandlung der longitudinale Strömungswiderstand längs der Hohlfasern der semipermeablen Membran des Dialysators auf der Blutseite von Bedeutung, der nachfolgend als Strömungswiderstand des Dialysators bezeichnet wird. Es ist bekannt, dass die Dämpfung von Druckpulsen längs der Hohlfasern der Membran des Dialysators mit dem Verhältnis der Amplituden der spektralen Anteile der ersten und zweiten Harmonischen zur Grundschwingung im Zusammenhang steht (WO 2008/135193 A1).

Die DE 600 37 408 T2 beschreibt eine Hämodialysevorrichtung, bei der die Substitutionsrate, mit der Substituat stromauf oder stromab des Dialysators dem extrakorporalen Blutkreislauf zugeführt wird, in Abhängigkeit von dem Transmembrandruck, dem Hämatokrit und der Blutdichte eingestellt wird.

Die DE 600 30 460 T2 beschreibt eine Dialysevorrichtung, bei der eine Regelung der Substituatrate erfolgt, um bestimmte Behandlungsziele zu erreichen, wobei die Koagulation des Filters vermieden werden soll. Hierzu wird die Förderarte der Substituatpumpe so eingestellt, dass die Parameter, die durch den Widerstand des Filters beeinflusst werden, auf ihren normalen Funktionswerten gehalten werden. Zur Bestimmung dieser Parameter wir insbesondere eine Druckmessung im Flüssigkeitssystem mit mehreren Drucksensoren vorgeschlagen.

Aus der WO 2008/135193 A1 ist bekannt, eine für den Strömungswiderstand des Dialysators charakteristische Größe zu bestimmen. Die Bestimmung dieser Größe soll aber nur zur Überwachung der Blutbehandlung dienen. Insbesondere soll eine für die Aufrechterhaltung des Blutflusses im extrakorporalen Kreislauf oder die Reinigungsleistung des Dialysators aussagekräftige Größe ermittelt werden. Die Veränderung der Substituatrate erfolgt nur für die Durchführung der eigentlichen Messung zur Bestimmung des Strömungswiderstandes.

Der Erfindung liegt die Aufgabe zugrunde, eine Vorrichtung zur extrakorporalen Blutbehandlung mit einer verbesserten Regelung der Substituatrate zu schaffen.

Die Lösung dieser Aufgabe erfolgt erfindungsgemäß mit den Merkmalen des Patentanspruchs 1. Eine vorteilhafte Ausführungsform der Erfindung ist Gegenstand des Unteranspruchs.

Die erfindungsgemäße Vorrichtung beruht darauf, dass die Regelung der Zufuhr von Substituat bei der extrakorporalen Blutbehandlung in Abhängigkeit von der rheologischen Belastung des Dialysators erfolgt. Dabei ist zu berücksichtigen, dass die Substituatrate keine unabhängige Größe ist, die nur in Abhängigkeit von der rheologischen Belastung des Dialysators geregelt werden kann, da die Substituatrate in einem Zusammenhang mit der Ultrafiltrationsrate steht. Die erfindungsgemäße Vorrichtung stellt daher darauf ab, von einer vorgegebenen Substituatrate auszugehen, mit der dem Patienten unter Berücksichtigung einer bestimmten Ultrafiltrationsrate Substituat zugeführt wird, wobei die vorgegebene Substituatrate in Abhängigkeit von der rheologischen Belastung des Dialysators erhöht oder verringert wird.

Die rheologische Belastung des Dialysators wird zur Regelung der Zufuhr von Substituat während der extrakorporalen Blutbehandlung bestimmt und die Substituatrate entsprechend der Belastung angehoben oder reduziert. Die Vorgabe von Dialysatorparametern oder Blutparametern ist nicht mehr notwendig. Selbst die Unterscheidung zwischen Prädilution oder Postdilution ist obsolet.

Die rheologische Belastung des Dialysators wird auf der Grundlage des Transmembrandrucks oder einer mit dem Transmembrandruck korrelierenden Größe und des Strömungswiderstands bestimmt, wobei der Transmembrandruck oder die mit dem Transmembrandruck korrelierende Größe und der Strömungswiderstand während der extrakorporalen Blutbehandlung ermittelt werden. Dabei ist unerheblich, wie der Transmembrandruck und der Strömungswiderstand gemessen werden. Allein entscheidend ist, dass der Transmembrandruck und der Strömungswiderstand bzw. von dem Transmembrandruck abgeleitete Größen für die weitere Auswertung zur Verfügung stehen, um die Zufuhr von

Substituat in Abhängigkeit von Transmembrandruck und Strömungswiderstand regeln zu können.

Eine bevorzugte Ausführungsform der Erfindung sieht vor, zur Bewertung des Transmembrandrucks oder der mit dem Transmembrandruck korrelierenden Größe eine erste Bewertungsgröße zu ermitteln und zur Bewertung des Strömungswiderstands eine zweite Bewertungsgröße zu ermitteln. Beide ermittelten Bewertungsgrößen bilden dann ein Bewertungspaar, das für die rheologische Belastung des Dialysators charakteristisch ist. Vorzugsweise werden der Transmembrandruck und der Strömungswiderstand innerhalb eines Bewertungsrahmens von 0-100 % bewertet. Mit dem Bewertungspaar wird die Rheologie am Dialysator vollständig beschrieben.

Bei einer bevorzugten Ausführungsform ist die Bewertung des Dialysators innerhalb des Bewertungsrahmens Eingangsparameter einer 2-dimensionalen Matrix, welche jedem Bewertungspaar (Prioritätenpaar) einen Wert zuweist, welcher der erforderlichen Änderung der Substituatsrate entspricht.

Jedem Bewertungspaar einer Vielzahl von die rheologische Belastung des Dialysators charakterisierenden Bewertungspaaren wird ein bestimmter Wert für den Betrag zugeordnet, um den die Substituatrate von einem vorgegebenen Wert erhöht oder verringert wird. Diese Zuordnung von Bewertungspaar und Betrag der Änderung der Substituatsrate kann in einem Speicher abgelegt werden. Damit steht für die unterschiedlichen Bewertungspaare jeweils der Wert zur Verfügung, um den die vorgegebene Substituatrate verändert wird.

Eine besonders bevorzugte Ausführungsform der Erfindung sieht vor, dass zur Bestimmung des Strömungswiderstands Druckpulse im extrakorporalen Blutkreislauf stromauf des Dialysators erzeugt und stromab des Dialysators gemessen werden, und dass das stromab des Dialysators gemessene Drucksignal spektral in eine Grundschwingung und mindestens einer Harmonische zerlegt wird. Der Strömungswiderstand wird dann auf der Grundlage des Verhältnisses der Grundschwingung und der mindestens einen Harmonischen bestimmt. Vorzugsweise wird das gemessene Drucksignal in eine Grundschwingung und zwei Harmonische zerlegt. Dieses Verfahren hat den Vorteil, dass der Druck im extrakorporalen Blutkreislauf nur stromab des Dialysators gemessen werden braucht. Als Druckpulse können die Druckpulse gemessen werden, die von der im extrakorporalen Blutkreislauf stromauf des Dialysators angeordneten Blutpumpe erzeugt werden, bei der es sich im Allgemeinen um eine okkludierende Schlauchpumpe handelt.

Die erfindungsgemäße Vorrichtung kann auf die ohnehin in einer extrakorporalen Blutbehandlungsvorrichtung im Allgemeinen vorhandene Sensorik zurückgreifen. Die Auswertung der Daten kann dabei in der zentralen Steuer- und Recheneinheit erfolgen, die ohnehin in der extrakorporalen Blutbehandlungsvorrichtung vorhanden ist. Daher lassen sich die erfindungsgemäße Vorrichtung und das erfindungsgemäße Verfahren ohne größeren konstruktiven Aufwand implementieren.

Im Folgenden wird ein Ausführungsbeispiel der Erfindung unter Bezugnahme auf die Zeichnungen im Einzelnen beschrieben:

Es zeigen:

Fig. 1    die wesentlichen Komponenten einer erfindungsgemäßen extrakorporalen Blutbehandlungsvorrichtung in vereinfachter schematischer Darstellung und

Fig. 2    eine Matrix, die jedem für die rheologische Belastung des Dialysators charakteristischen Bewertungspaar einen Wert zuweist, welcher der erforderlichen Änderung der Substituatrate entspricht.

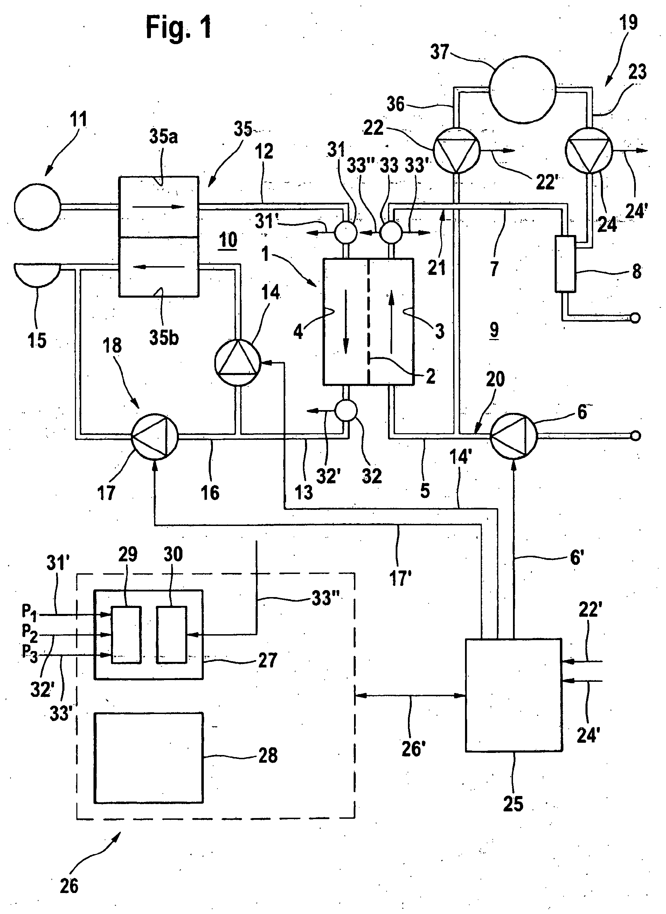

Fig. 1 zeigt die wesentlichen Komponenten der erfindungsgemäßen Blutbehandlungsvorrichtung, bei der es sich um eine Hämo(dia)filtrationsvorrichtung handelt, die einen Dialysator (Filter) 1 aufweist, der durch eine semipermeable Membran 2 in eine Blutkammer 3 und eine Dialysierflüssigkeitskammer 4 getrennt ist. Der Einlass der Blutkammer 3 ist mit einem Ende einer Blutzuführleitung 5 verbunden, in die eine Blutpumpe 6, insbesondere eine Druckpulse erzeugende Rollenpumpe, geschaltet ist, während der Auslass der Blutkammer mit einem Ende einer Blutabführleitung 7 verbunden ist, in die eine Tropfkammer 8 geschaltet ist. Blutzuführ- und -abführleitung 5, 7 bilden mit der Blutkammer 3 des Dialysators den extrakorporalen Blutkreislauf 9 der Hämodiafiltrationsvorrichtung. Bei der Blutzuführ- und -abführleitung 5, 7 handelt es sich um Schlauchleitungen eines in die Hämodiafiltrationsvorrichtung eingelegten Schlauchsets (Disposable).

Das Dialysierflüssigkeitssystem 10 der Hämodiafiltrationsvorrichtung umfasst eine Einrichtung 11 zur Bereitstellung von Dialysierflüssigkeit, die über den ersten Abschnitt einer Dialysierflüssigkeitszuführleitung 12 mit dem Einlass der ersten Bilanzkammerhälfte 35a einer Bilanziereinrichtung 35 verbunden ist. Der zweite Abschnitt der Dialysierflüssigkeitszuführleitung 12 verbindet den Auslass der ersten Bilanzkammerhälfte 35a mit dem Einlass der Dialysierflüssigkeitskammer 4. Der Auslass der Dialysierflüssigkeitskammer 4 ist über den ersten Abschnitt einer Dialysierflüssigkeitsabführleitung 13 mit dem Einlass der zweiten Bilanzkammerhälfte 35b verbunden. In den ersten Abschnitt der Dialysierflüssigkeitsabführleitung 13 ist eine Dialysierflüssigkeitspuinpe 14 geschaltet. Der Auslass der zweiten Bilanzkammerhälfte 35b ist über den zweiten Abschnitt der Dialysierflüssigkeitsabführleitung 13 mit einem Auslauf 15 verbunden. Stromauf der Dialysierflüssigkeitspumpe 14 zweigt von der Dialysierflüssigkeitsabführleitung 13 eine Ultrafiltratleitung 16 ab, die ebenfalls zu dem Auslauf 15 führt. In die Ultrafiltratleitung 16 ist eine Ultrafiltrationspumpe 17 geschaltet. Die Bilanziereinrichtung 35 besteht bei handelsüblichen Geräten aus zwei parallelen Bilanzkammern, die anti-zyklisch betrieben werden. Aus Gründen der Vereinfachung ist hier nur eine Bilanzkammer dargestellt.

Während der Dialysebehandlung wird die Blutkammer 3 von dem Blut des Patienten und die Dialysierflüssigkeitskammer 4 des Dialysators von der Dialysierflüssigkeit durchströmt. Die Bilanziereinrichtung 35 stellt sicher, dass nur so viel Dialysierflüssigkeit über die Dialysierflüssigkeitszuführleitung zufließen kann, wie Dialysierflüssigkeit über die Dialysierflüssigkeitsabführleitung abfließen kann. Mit der Ultrafiltrationspumpe 17 kann dem Patienten eine vorgegebene Menge an Flüssigkeit (Ultrafiltrat) mit einer vorgegebenen Ultrafiltrationsrate entzogen werden. Die Ultrafiltrationspumpe 17 ist somit Teil einer Einrichtung zum Entziehen von Flüssigkeit aus dem im extrakorporalen Kreislauf 9 strömenden Blut durch die Membran 2 des Dialysators 1, die als Ultrafiltrationseinrichtung 18 bezeichnet wird.

Um dem Patienten die Flüssigkeit wieder zuzuführen, verfügt die Hämodiafiltrationsvorrichtung über eine Substitutionseinrichtung 19, mit der eine Substitutionsflüssigkeit (Substituat) dem Blut zugeführt werden kann, das durch den arteriellen Zweig 20 (Prädilution) und/oder den venösen Zweig 21 (Postdilution) des extrakorporalen Blutkreislaufs 9 strömt. Die Substitutionseinrichtung 19 weist eine Einrichtung 37 zur Bereitstellung von Substituat auf, von der eine erste Substituatleitung 36, in die eine erste Substituatpumpe 22 geschaltet ist, zu dem Abschnitt der Blutzuführleitung 5 zwischen Blutpumpe 6 und Blutkammer 3 führt. Eine zweite Substituatleitung 23, in die eine zweite Substituatpumpe 24 geschaltet ist, führt von der Einrichtung 37 zur Bereitstellung von Substituat zu der Tropfkammer 8. Wenn die Hämodiafiltrationsvorrichtung nur mit Postdilution bzw. Prädilution betrieben werden soll, kann die eine oder andere Substituatpumpe zusammen mit der jeweiligen Substituatleitung entfallen.

Darüber hinaus weist die Hämodiafiltrationsvorrichtung eine zentrale Steuer- und Recheneinheit 25 auf, die über Steuerleitungen 6', 14', 17', 22', 24' mit der Blutpumpe 6, der Dialysierflüssigkeitspumpe 14, der Ultrafiltrationspumpe 17 sowie der ersten und zweiten Substituatpumpe 22, 24 verbunden ist.

Die extrakorporale Blutbehandlungsvorrichtung verfügt über eine Einrichtung 26 zum Regeln der Zufuhr von Substituat, die in Fig. 1 in gestrichelten Linien dargestellt ist. Die Einrichtung 26 zum Regeln der Zufuhr von Substituat ist in Fig. 1 als getrennte Einrichtung dargestellt. Sie kann aber auch Bestandteil der zentralen Steuer- und Recheneinheit 25 sein. Die Einrichtung 26 zum Regeln der Zufuhr von Substituat ist mit der zentralen Steuer- und Recheneinheit 25 über eine Datenleitung 26' verbunden, so dass die Regeleinrichtung mit der Steuereinheit Daten austauschen kann, insbesondere die Substituatpumpen 22, 24 zur Einstellung der Substituatrate Q entsprechend ansteuern kann.

Die Einrichtung 26 zum Regeln der Zufuhr von Substituat weist Mittel 27 zum Bestimmen der rheologischen Belastung des Dialysators und Mittel 28 zum Regeln der Substituatrate auf.

Die Mittel 27 zum Bestimmen der rheologischen Belastung des Dialysators verfügen wiederum über Mittel 29 zum Bestimmen des Transmembrandrucks am Dialysator oder einer mit dem Transmembrandrucks korrelierenden Größe und Mittel 30 zum Bestimmen des Strömungswiderstands des Dialysators. Dabei wird unter dem Strömungswiderstand des Dialysators der longitudinale Strömungswiderand längs der Hohlfaser der semipermeablen Membran 2 des Dialysators 1 auf der Blutseite verstanden.

Die Mittel 29 zum Bestimmen des Transmembrandrucks (TMP) können unterschiedlich ausgebildet sein. Zur Bestimmung des Transmembrandrucks kann beispielsweise die in der EP 0 212 127 A1 beschriebene Messvorrichtung verwendet werden. Bei dem vorliegenden Ausführungsbeispiel umfassen die Mittel 27 zum Bestimmen des Transmembrandrucks einen in der Dialysierflüssigkeitszuführleitung 12 stromauf der Dialysierflüssigkeitskammer 4 des Dialysators 1 angeordneten ersten Drucksensor 31, einen in der Dialysierflüssigkeitsabführleitung 16 stromab der Dialysierflüssigkeitskammer des Dialysators angeordneten zweiten Drucksensor 32 und einen in der Blutrückführleitung 21 stromab der Blutkammer 3 des Dialysators 1 angeordneten dritten Drucksensor 33. Die Drucksensoren 31, 32, 33, sind über Datenleitungen 31', 32', 33' mit den Mitteln 29 zum Bestimmen des Transmembrandrucks verbunden. Mit den Drucksensoren 31 und 32 wird im Dialysierflüssigkeitssystem 10 der Druck $P_1$ stromauf und der Druck $P_2$ stromab der Dialysierflüssigkeitskammer und mit dem Drucksensor 33 im extrakorporalen Blutkreislauf 9 der Druck $P_3$ stromab der Blutkammer gemessen.

Die Mittel 29 zum Bestimmen des Transmembrandrucks TMP verfügen über eine geeignete Recheneinheit, die den Transmembrandruck nach der folgenden Gleichung berechnet:

$$TMP = P_3 - \frac{P_1 + P_2}{2}$$

Der ermittelte Wert für den Transmembrandruck TMP wird wie folgt bewertet. Zur Bewertung des Transmembrandrucks TMP wird nach der folgenden Gleichung aus dem gemessenen Wert für den Transmembrandruck TMP und einem vorgegebenen unteren Grenzwert für den Transmembrandruck $TMP_{LIMIT\_LOWER}$ und einem vorgegebenen oberen Grenzwert für den Transmembrandruck $TMP_{LIMIT\_UPPER}$ sowie einem vorgegebenen Wertebereich für den Transmembrandruck $TMP_{LIMIT\_RANGE}$ eine erste Bewertungsgröße HEMO-Priorität berechnet. Dabei werden die Parameter $TMP_{LIMIT\_LOWER}$, $TMP_{LIMIT\_UPPER}$ und $TMP_{LIMIT\_RANGE}$ empirisch ermittelt.

$$\text{HEMO\_Priorität} = ((TMP - TMP_{LIMIT\_LOWER}) / TMP_{LIMIT\_RANGE}) * 100\%$$

wobei $TMP_{LIMIT\_RANGE} = TMP_{LIMIT\_UPPER} - TMP_{LIMIT\_LOWER}$

Neben dem Transmembrandruck TMP wird zur Bestimmung der rheologischen Belastung des Dialysators 1 der Strömungswiderstand des Dialysators ermittelt.

Die Mittel 30 zum Bestimmen des Strömungswiderstands verfügen über Mittel zum Messen von Druckpulsen, die sich in longitudinaler Richtung über die Hohlfasern der semipermeablen Membran des Dialysators auf der Blutseite ausbreiten. Die Druckpulse werden von der Blutpumpe 6 erzeugt, bei der es sich um eine okkludierende Schlauchpumpe, insbesondere Rollenpumpe handelt. Bei dem vorliegenden Ausführungsbeispiel wird der stromab der Blutkammer 3 in der Blutrückführleitung 21 angeordnete Drucksensor 33 zum Messen der von der Blutpumpe 6 erzeugten Druckpulse verwendet. Daher führt eine zweite Datenleitung 33" von dem Drucksensor 33 zu den Mitteln 30 zum Bestimmen des Strömungswiderstands. Zum Bestimmen des Strömungswiderstands wird das mit dem Drucksensor 33 gemessene Drucksignal spektral in eine Grundschwingung $G_0$ und die erste und zweite-Harmonische $H_1$ und $H_2$ zerlegt, da die Dämpfung der Druckpulse längs der Hohlfasern im Verhältnis der Amplituden der spektralen Anteile der ersten und zweiten Harmonischen $H_1$ und $H_2$ zur Grundschwingung Go im Zusammenhang stehen. Der theoretische Zusammenhang ist in der WO 2008/135193 A1 beschrieben.

Zur Regelung der Substituatrate wird auch der Strömungswiderstand wie folgt bewertet. Nach der folgenden Gleichung wird aus der Grundschwingung $G_0$ und der ersten und zweiten Harmonischen $H_1$ und $H_2$ sowie den empirisch festgelegten Parametern $K_{1,2}$, $M_{1,2}$ und $\alpha$ nach der folgenden Gleichung

$$BLKD\_Priorität = \alpha \cdot \left( \frac{G_0 / H_1 - K_1}{2M_1} + \frac{G_0 / H_2 - K_2}{2M_2} \right)$$

eine zweite Bewertungsgröße BLKD_Priorität berechnet.

Die erste und zweite Bewertungsgröße bilden ein Bewertungspaar (Hemo_Priorität/BLKD_Priorität), das für die rheologische Belastung des Dialysators charakteristisch ist.

Die Frequenz der Grundschwingung der Druckpulse ergibt sich aus der Ansteuerung der Blutpumpe 6. Damit sind auch die Frequenzen der ersten und zweiten Harmonischen der Grundschwingung bekannt. Die Zerlegung des kontinuierlichen Drucksignals in seine spektralen Anteile erfolgt bevorzugt mit einer Fouriertransformation, besonders bevorzugt durch Digitalisieren der Messwerte des Drucksensors 33 mit diskreter Fouriertransformation, die in einer geeigneten Recheneinheit durchgeführt wird.

Der Vorteil der Analyse der Druckpulse zur Bestimmung des Strömungswiderstands liegt darin, dass nur ein Sensor stromab des Dialysators erforderlich ist. Ein Sensor stromauf des Dialysators ist hingegen nicht erforderlich. Es ist aber auch möglich, den Strömungswiderstand durch Messungen mit vier Drucksensoren stromauf und stromab des Dialysators auf der Blut- und Dialysierflüssigkeitsseite zu bestimmen. Auch ist es möglich; den Strömungswiderstand durch eine Messung mit zwei Drucksensoren stromab des Dialysators auf der Blut- und Dialysierflüssigkeitsseite näherungsweise zu bestimmen, in dem die Drücke stromauf des Dialysators auf der Blut- und Dialysierflüssigkeitsseite auf der Grundlage von Betriebsparametern abgeschätzt werden.

Da die rheologische Belastung des Dialysators sowohl auf der Grundlage des Transmembrandrucks als auch des Strömungswiderstands bestimmt wird, ist die Messung des Transmembrandrucks mit nur zwei oder drei Drucksensoren anstelle der bekannten Messung mit vier Drucksensoren ausreichend, obwohl sich die Zwei- und die Drei-Punktmessung des Transmembrandrucks in der Praxis nicht immer als zuverlässig erwiesen haben, da es bei der Zwei- und der Drei-Punktmessung zu einem unstetigen Verhalten im Bereich besonders hoher Transmembrandrücke kommen kann.

Bei dem vorliegenden Ausführungsbeispiel werden der Transmembrandruck und der Strömungswiderstands derart bewertet, dass die Bewertungsgrößen innerhalb eines Bewertungsrahmens von 0 bis 100 % skalieren. Die rheologische Belastung des Dialysators lässt sich als Punkt in einem zweidimensionalen Koordinatensystem vollständig beschreiben. Die Regelung der Substituatrate basiert darauf, die rheologische Belastung innerhalb eines Zielbereichs der Matrix zu halten. Die Regelung erfolgt unabhängig davon, ob eine Post- oder Prädilution vorliegt.

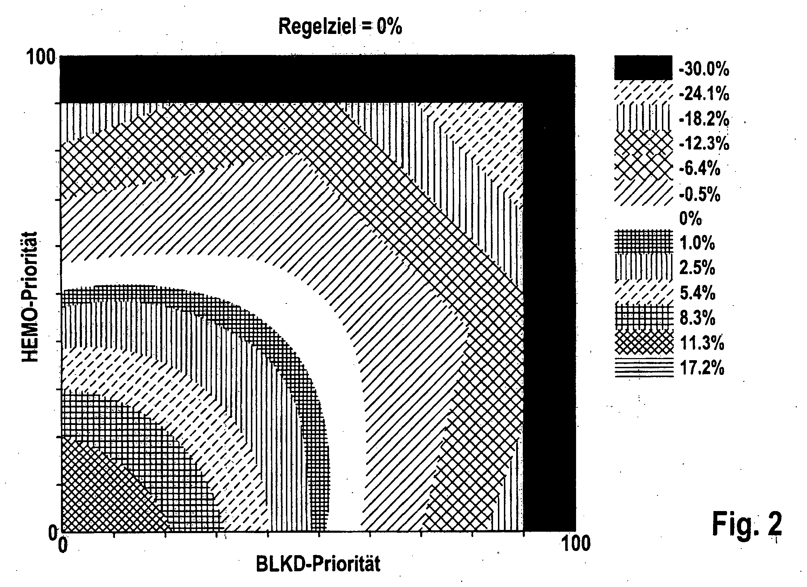

Fig. 2 zeigt die zweidimensionale Matrix, welche jedem Bewertungspaar (Prioritätenpaar) einen Wert zuweist, welcher der erforderlichen Änderung der Substituatrate entspricht. Folglich wird jedem Wertepaar, das in der Matrix abgelegt ist, ein bestimmter Wert für den Betrag der Änderung der vorgegebenen Substituatrate zugeordnet. Innerhalb der Matrix existiert eine Solllinie (Wertebereich), welche die Bewertungspaare miteinander verbindet, die der angestrebten Dialysatorbelastung entsprechen. Diese Solllinie ist eine Linie, die sich mathematisch aus der Verbindung einer zu den Prioritäten linear verlaufenden Linie und einer um das Prioritätenpaar (0,0) verlaufenden Kreislinie zusammensetzt. Liegt das Prioritätenpaar auf der Solllinie, so bleibt die Substituatrate unverändert. Die Solllinie (Wertebereich) ist in Fig. 1 als nicht schraffierter Bereich gekennzeichnet. Der Betrag der Änderung der Substituatrate ist in Fig. 1 durch die Dichte der Schraffierung dargestellt. Die Skala rechts in Fig. 1 ordnet den schraffierten Bereichen im Koordinatensystem links entsprechende Änderungen der Substituatrate zu. Regelziel ist hierbei der nicht schraffierte Bereich (0 %), was gleichbedeutend mit unveränderter Substituatgabe ist.

In der Einrichtung 26 zum Regeln der Zufuhr von Substituat wird aus der Matrix die erforderliche Substituaträtenänderung $\alpha$ bestimmt. Die neu einzustellende Substituatrate $Q_{sub,\,neu}$ wird wie folgt berechnet:

$$Q_{sub,neu} = Q_{sub,alt}(1+\alpha)$$

Für die extrakorporale Blutbehandlung wird eine bestimmte Ultrafiltrationsrate vorgegeben, die mit der Ultrafiltrationseinrichtung 18 eingestellt wird. Weiterhin wird vorgegeben, ob dem Patienten Flüssigkeit zugeführt oder entzogen oder weder Flüssigkeit zugeführt noch entzogen werden soll. Wenn beispielsweise dem Patienten Flüssigkeit entzogen werden soll, gibt die zentrale Steuer- und Recheneinheit 25 eine bestimmte Substituatrate vor. Diese Substituatrate ist dann derart bemessen, dass dem extrakorporalen Blutkreislauf weniger Substituat zugeführt wird, als über die Membran 2 des Dialysators 1 mit der Ultrafiltrationseinrichtung 18 Flüssigkeit entzogen wird. Diese vorgegebene Substituatrate wird mit der Einrichtung 26 zum Regeln der Zufuhr von Substituat nach dem oben beschriebenen Verfahren erhöht oder verringert. Damit wird die extrakorporale Blutbehandlung unter optimalen Bedingungen für den Dialysator durchgeführt.

Die Regelung der Substituatzugabe sieht nicht nur eine Veränderung der Substituatrate vor, sondern auch eine Verteilung der Zufuhr von Substituat stromauf und stromab des Dialysators (Prädilution und Postdilution). Bei der Zufuhr von Substituat sowohl stromauf als auch stromab des Dialysators wird die Gesamtdilutionsmenge auf Post- und Prädilution nach der Vorgabe durch die Matrix verändert. Als Bestimmungsparameter für eine

Änderungsanweisung für die Gesamtdilutionsmenge dient hierbei der Abstand des für die rheologische Belastung des Dialysators charakteristischen Wertepaars im Koordinatensystem vom Koordinatenursprung (0,0) und der Winkel zwischen der gedachten Linie, die durch den Koordinatenursprung (0,0) und das charakteristische Bewertungspaar verläuft, und der X-Achse oder alternativ der Y-Achse. Die Einrichtung 26 zum Regeln der Zufuhr von Substituat stellt dann zusammen mit der zentralen Steuer- und Recheneinheit 25 entsprechend des ermittelten Abstands und Winkels die Flussraten der Substituatpumpen 22 und 30 entsprechend ein.

## Patentansprüche

1. Vorrichtung zur extrakorporalen Blutbehandlung mit

einem durch eine semipermeable Membran (2) in eine Blutkammer (3) und eine Dialysierflüssigkeitskammer (4) unterteilten Dialysator (1), wobei die Blutkammer Teil eines extrakorporalen Blutkreislaufs (9) und die Dialysierflüssigkeitskammer Teil eines Dialysierflüssigkeitssystems (10) ist;
einer Einrichtung (19) zum Zuführen von Substituat mit einer vorgegebenen Substituatrate zu dem extrakorporalen Blutkreislauf (9) und
einer Einrichtung (26) zum Regeln der Zufuhr von Substituat,
**dadurch gekennzeichnet, dass**
die Einrichtung (26) zum Regeln der Zufuhr von Substituat aufweist:

Mittel (27) zum Bestimmen der rheologischen Belastung des Dialysators, die Mittel (29) zum Bestimmen des Transmembrandrucks oder einer mit dem Transmembrandruck korrelierenden Größe, die von dem Transmembrandruck abhängig ist, und Mittel zum Bestimmen des Strömungswiderstands des Dialysators aufweisen, wobei die Mittel zum Bestimmen (27) der rheologischen Belastung des Dialysators derart ausgebildet sind, dass die rheologische Belastung des Dialysators auf der Grundlage des Transmembrandrucks oder der mit dem Transmembrandruck korrelierenden und von dem Transmembrandruck abhängigen Größe und des Strömungswiderstands bestimmt wird, und

Mittel (28) zum Regeln der Substituatrate, die derart ausgebildet sind, dass die Substituatrate in Abhängigkeit von der rheologischen Belastung des Dialysators geregelt wird.

2. Vorrichtung nach Anspruch 1, **dadurch gekennzeichnet, dass** die Mittel (27) zur Bestimmung des Transmembrandrucks Mittel (33) zum Messen des Drucks im extrakorporalen Blutkreislauf und im Dialysierflüssigkeitssystem aufweisen.

## Claims

1. Device for extracorporeal blood treatment, comprising

   a dialyser (1) which is divided into a blood chamber (3) and a dialysate chamber (4) by a semipermeable membrane (2), the blood chamber being part of an extracorporeal blood circuit (9) and the dialysate chamber being part of a dialysate system (10);
   an apparatus (19) for supplying substituate to the extracorporeal blood circuit (9) at a predetermined substituate rate, and
   an apparatus (26) for controlling the substituate supply,
   **characterised in that**
   the apparatus (26) for controlling the substituate supply comprises:

   means (27) for determining the rheological load of the dialyser, the means (27) for determining the rheological load of the dialyser comprising means (29) for determining the transmembrane pressure or a variable which correlates with the transmembrane pressure and is dependent on the transmembrane pressure, and means for determining the flow resistance of the dialyser, the means (27) for determining the rheological load of the dialyser being designed such that the rheological load of the dialyser is determined on the basis of the transmembrane pressure or the variable which correlates with the transmembrane pressure and is dependent on the transmembrane pressure, and on the basis of the flow resistance, and
   means (28) for controlling the substituate rate, which are designed such that the substituate rate is controlled depending on the rheological load of the dialyser.

2. Device according to claim 1, **characterised in that** the means (27) for determining the transmembrane pressure comprise means (33) for measuring the pressure in the extracorporeal blood circuit and in the dialysate system.

## Revendications

1. Dispositif de traitement extracorporel du sang comprenant

   un dialyseur (1) séparé par une membrane semi-perméable (2) en une chambre de sang (3) et une chambre de liquide de dialyse (4), la chambre de sang étant partie intégrante d'une circulation sanguine extracorporelle (9) et la chambre de liquide de dialyse étant partie intégrante d'un système de liquide de dialyse (10) ;
   un dispositif (19) d'alimentation de la circulation sanguine extracorporelle (9) en solution de substitution avec un débit de solution de substitution prédéterminé et un dispositif (26) de régulation de l'alimentation en solution de substitution, **caractérisé en ce que**
   le dispositif (26) de régulation de l'alimentation en solution de substitution présente :

   des moyens (27) de détermination de la charge rhéologique du dialyseur, qui présentent des moyens (29) de détermination de la pression transmembranaire ou d'une grandeur en corrélation avec la pression transmembranaire, qui dépend de la pression transmembranaire, et des moyens de détermination de la résistance à l'écoulement du dialyseur, les moyens de détermination (27) de la charge rhéologique du dialyseur étant façonnés de telle manière que la charge rhéologique du dialyseur est déterminée sur la base de la pression transmembranaire ou de la grandeur en corrélation avec la pression transmembranaire et dépendant de la pression transmembranaire et de la résistance à l'écoulement, et

des moyens (28) de régulation du débit de solution de substitution, qui sont façonnés de telle manière que le débit de solution de substitution est régulé en fonction de la charge rhéologique du dialyseur.

2. Dispositif selon la revendication 1, **caractérisé en ce que** les moyens (27) de détermination de la pression transmembranaire présentent des moyens (33) de mesure de la pression dans la circulation sanguine extracorporelle et dans le système de liquide de dialyse.

Fig. 1

Regelziel = 0%

Fig. 2

**EP 2 504 044 B2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0212127 A1
- WO 2009080258 A1
- WO 2008135193 A1
- DE 60037408 T2
- DE 60030460 T2